# DEMANDE DE BREVET EUROPEEN

(11) **EP 4 450 048 A1**
(43) Date de publication de la demande: **23.10.2024**
(21) Numéro de dépôt: 23305590.4
(22) Date de dépôt: 18.04.2023
(51) Int. Cl.: A61K 8/02, A61K 8/29, A61K 8/365, A61K 8/44, A61K 8/73, A61K 8/9728, A61Q 1/02, A61Q 17/04, A61Q 19/00

(54) **COMPOSITION À BASE DE CHITOSAN ET DE PIGMENT MINÉRAL NANOMÉTRIQUE AYANT SUBI UN TRAITEMENT DE SURFACE SPÉCIFIQUE**

(71) Demandeur: L'OREAL, 75008 Paris (FR)
(72) Inventeur: LE VERGE, Danielle, 94550 CHEVILLY LARUE (FR); MOINE, Toni, 94550 CHEVILLY LARUE (FR); KUSINA, Christophe, 94550 CHEVILLY LARUE (FR)
(74) Mandataire: Lavoix

(57) **Abrégé**

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment minéral traité en surface, ledit pigment présentant une taille inférieure ou égale à 100 nm.

Elle se rapporte également à un procédé de maquillage et/ou de soin de la peau et/ou des phanères, dans lequel on applique la composition selon l'invention sur la peau et/ou les phanères.

## Description

La présente invention concerne une composition cosmétique comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment minéral traité en surface, ledit pigment présentant une taille inférieure ou égale à 100 nm.

Elle se rapporte également à un procédé de maquillage et/ou de soin de la peau et/ou des phanères, dans lequel on applique la composition selon l'invention sur la peau et/ou les phanères.

Il existe de nombreuses compositions cosmétiques pour lesquelles des propriétés de tenue du film déposé, après application sur les matières kératiniques, sont souhaitées. On peut citer par exemple les rouges à lèvres ou les vernis à ongles. Afin d'obtenir un tel résultat, il est possible d'associer des matières premières particulières, notamment des agents filmogènes. Cependant, la présence de tels agents pour la tenue du film sur les matières kératiniques peut conduire à des compositions desséchantes. Par ailleurs, les agents filmogènes couramment utilisés sont généralement de nature pétrochimique, comme les silicones ou les polymères acryliques.

Par ailleurs, on cherche souvent à obtenir des compositions couvrantes.

Le formulateur est donc à la recherche de matières premières et/ou de systèmes permettant d'obtenir des compositions fluides (i.e. qui s'écoulent) dont le dépôt est couvrant, homogène et résistant (i.e. présentant une bonne tenue), en particulier présentant une bonne tenue à l'eau.

Par ailleurs, la formulation de produits cosmétiques respectueux de l'environnement, c'est-à-dire dont la conception et le développement tiennent compte des enjeux environnementaux, devient une préoccupation majeure pour contribuer à relever les défis planétaires.

Il se révèle donc essentiel de proposer des compositions et/ou des procédés de préparation et/ou des ingrédients plus durables permettant ainsi de répondre à ces enjeux environnementaux.

Dans ce contexte, il est important de développer de nouvelles compositions cosmétiques avec une meilleure empreinte carbone notamment en favorisant l'emploi de matières premières renouvelables et/ou avec un bon index de naturalité et/ou d'origine naturelle et plus particulièrement d'origine végétale tout en réduisant l'utilisation de composés d'origine pétrochimique.

La présente invention a pour but de proposer des compositions cosmétiques aqueuses présentant de bonnes propriétés maquillantes, notamment en termes de couvrance et d'homogénéité, mais aussi ayant une bonne tenue, en particulier ayant une bonne tenue/résistance à l'eau.

Après application, ces compositions laissent un dépôt filmogène couvrant et homogène, qui a une bonne tenue à l'usure. Les films formés sont adhésifs et cohésifs, et présentent une résistance améliorée à l'eau.

Par ailleurs, le traitement de surface du pigment minéral, ledit pigment présentant une taille inférieure ou égale à 100 nm, permet d'améliorer le facteur de protection solaire de la composition obtenue.

Ces compositions comprennent également des ingrédients durables, permettant ainsi de répondre aux enjeux environnementaux.

La présente invention a donc pour objet une composition cosmétique, notamment de maquillage et/ou de soin de la peau et/ou des lèvres, en particulier pour le soin de la peau, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment minéral traité en surface, ledit pigment présentant une taille inférieure ou égale à 100 nm.

Par « physiologiquement acceptable », on entend un milieu compatible avec les matières kératiniques.

Elle a également pour objet un procédé de maquillage et/ou de soin de la peau et/ou des phanères, dans lequel on applique la composition selon l'invention sur la peau et/ou les phanères.

De préférence, la composition selon l'invention est substantiellement exempte de polymère (méth)acrylique. De préférence, la composition selon l'invention est substantiellement exempte de silicone.

Par « substantiellement exempte de polymère (méth)acrylique », on entend que la composition comprend moins de 1% en poids par rapport au poids total de la composition, de préférence moins de 0,5% en poids, de préférence moins de 0,3% en poids, préférentiellement 0,1% en poids de polymère (méth)acrylique. De préférence, la composition est totalement exempte de polymère (méth)acrylique. Par polymère (méth)acrylique, on entend un polymère comprenant au moins un monomère d'acide acrylique ou au moins un monomère d'acide méthacrylique.

Par « substantiellement exempte de silicone », on entend que la composition comprend moins de 1 % en poids par rapport au poids total de la composition, de préférence moins de 0,5% en poids, de préférence moins de 0,3% en poids, préférentiellement 0,1% en poids de silicone. De préférence, la composition est totalement exempte de silicone. Par silicone, on entend tout composé siliconé.

### Chitosan

La composition selon l'invention comprend au moins 0,01% en poids par rapport au poids total de la composition d'au moins un chitosan natif ayant un poids moléculaire strictement supérieur à 3000 Daltons (3 kDa).

La quantité de chitosan natif est également strictement inférieure à 15% en poids par rapport au poids total de la composition.

De préférence, le chitosan natif a un poids moléculaire supérieur ou égal à 10 kDa, de préférence supérieur ou égal à 15 kDa, de préférence supérieur ou égal à 20 kDa. De préférence, le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

Le chitosan (ou chitosane) est très peu répandu dans la nature. Il n'est signalé que dans les exosquelettes de certains insectes comme les reines des termites et dans les parois cellulaires d'une classe particulière de champignons, les zygomycètes.

Le chitosan est obtenu par désacétylation de la chitine. La chitine est un polysaccharide composé de plusieurs unités N-acétyl-D-glucosamine reliées entre elles par une liaison de type β (1,4).

La structure chimique idéale du chitosane est un enchaînement de monomères β-D-glucosamine reliés par une liaison glycosidique (1→4).

Par « chitosan » selon l'invention, on entend tout copolymère formé d'unités constitutives N-acétyl-D-glucosamine et D-glucosamine, dont le degré d'acétylation est inférieur à 90%. Le chitosan est constitué des unités sucres glucosamine (unités désacétylées) et d'unités N-acétyl-D-glucosamine (unités acétylées) reliées entre elles par des liaisons de type β (1,4) et constitue un polymère du type Poly(N-acetyl-D-glucosamine)-poly(D-glucosamine).

De préférence, le degré d'acétylation du chitosan est inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

Le degré d'acétylation est le pourcentage d'unités acétylées par rapport au nombre d'unités totales, il peut être déterminé par spectroscopie infrarouge à transformée de Fourier (IR-TF) ou par un titrage par une base forte.

Le chitosan de l'invention est de préférence un polysaccharide préparé à partir d'une origine fongique. Il est notamment extrait et purifié à partir de sources fongiques alimentaires ou biotechnologique sûres et abondantes tels que *Agaricus bisporus* ou *Aspergillus niger.*

Le chitosan de l'invention est de préférence issu du mycélium d'un champignon du type Ascomycète, et en particulier *d'Aspergillus niger* et/ou d'un champignon Basidiomycète, et en particulier *Lentinula edodes* (shiitake) et/ou *Agaricus bisporus.* De préférence le champignon est *Aspergillus niger.*

Le chitosan peut être d'origine OGM, mais de préférence est d'origine non OGM.

Le chitosan selon l'invention est natif, c'est-à-dire qu'il n'est pas modifié. Il ne contient notamment pas de modification chimique.

Une méthode de préparation du chitosan est celle décrite dans la demande WO03068824.

De préférence, le chitosan utilisé dans l'invention est sous forme de poudre. Il est notamment commercialisé par Kitozyme sous le nom Kiosmetine ou Kionutrime.

Le chitosan est de préférence présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

### Pigment minéral nanométrique traité en surface

La composition selon l'invention comprend au moins un pigment minéral traité en surface, ledit pigment présentant une taille inférieure ou égale à 100 nm (« pigment minéral nanométrique traité en surface »).

On entend par « pigments » des particules minérales, blanches ou colorées, insolubles dans un milieu aqueux, destinées à colorer la composition et/ou le dépôt résultant.

Les pigments traités peuvent être présents, dans la composition, en une teneur allant de de 1 à 70% en poids, de préférence de 1 à 60% en poids, de préférence de 1% à 50% en poids, de préférence de 3% à 45% en poids, par rapport au poids de la composition, de préférence de 4% à 30% en poids, de préférence de 6% à 20% en poids, de préférence de 6% à 15% en poids.

De préférence, le ratio pondéral entre le pigment minéral nanométrique traité en surface et le chitosan (i.e. ratio pondéral pigment : chitosan) est supérieur à 1, de préférence compris entre 1 et 30. De préférence, il est compris entre 1 et 25, préférentiellement entre 5 et 20.

### Pigments minéraux

Par « pigment minéral », on entend tout pigment qui répond à la définition de l'encyclopédie Ullmann dans le chapitre pigment inorganique.

La taille du pigment minéral utile dans le cadre de la présente invention est nanométrique, i.e. inférieure ou égale à 100 nm. Selon une forme particulière de l'invention, les pigments présentent une taille caractérisée par un D[50] inférieur à 100 nm, de préférence inférieur à 50 nm, de préférence compris entre 5 et 40 nm, de préférence compris entre 10 et 30 nm.

Les tailles sont mesurées par diffusion statique de la lumière au moyen d'un granulomètre commercial de type MasterSizer 3000^{®} de chez Malvern, permettant d'appréhender la répartition granulométrique de l'ensemble des particules sur une large gamme pouvant aller de 0,01 µm à 1000 µm. Les données sont traitées sur la base de la théorie classique de diffusion de Mie. Cette théorie est la plus adaptée pour des distributions de taille allant du submicronique au multi-micronique, elle permet de déterminer un diamètre « effectif » de particules. Cette théorie est notamment décrite dans l'ouvrage de Van de Hulst, H.C., « Light Scattering by Small Particles », Chapitres 9 et 10, Wiley, New York, 1957.

D[50] représente la taille maximale que présentent 50 % en volume des particules.

Tout pigment minéral présentant une taille nanométrique est utilisable dans la présente invention.

On peut citer, parmi les pigments minéraux utiles dans la présente invention, le dioxyde de titane, les oxydes de fer (noir, jaune ou rouge), les oxydes de zirconium ou de cérium, les oxydes de zinc ou de chrome, le violet de manganèse, le bleu outremer, l'hydrate de chrome et le bleu ferrique, les poudres métalliques comme la poudre d'aluminium et la poudre de cuivre.

De préférence, le pigment minéral est plus particulièrement le dioxyde de titane.

On peut également citer les nacres et/ou les pigments à effet optique différent d'un simple effet de teinte conventionnel (comme par exemple les pigments monochromatiques), dès lors que leur taille est nanométrique.

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques. On peut également citer, à titre d'exemple de nacres, le mica naturel recouvert d'oxyde de titane, d'oxyde de fer, de pigment naturel ou d'oxychlorure de bismuth. Les nacres peuvent plus particulièrement posséder une couleur ou un reflet jaune, rose, rouge, bronze, orangé, brun, or et/ou cuivré.

Parmi les pigments utilisables selon l'invention, on peut également citer ceux à effet optique différent d'un simple effet de teinte conventionnel, c'est-à-dire unifié et stabilisé tel que produit par les matières colorantes classiques, comme par exemple, les pigments monochromatiques.

Au sens de l'invention, « stabilisé » signifie dénué d'effet de variabilité de la couleur avec l'angle d'observation ou encore en réponse à un changement de température.

### Agent de traitement de surface

L'agent de traitement de surface selon l'invention est un agent hydrophobe. Il est choisi de préférence parmi les acides gras, les sels métalliques et leurs mélanges.

### Acides gras

On entend par « acide gras » un acide carboxylique à chaîne aliphatique comprenant 8 à 30 atomes de carbone, de préférence, de 12 à 24 atomes de carbone et encore plus préférentiellement, de 16 à 18 atomes de carbone. L'acide gras peut être liquide ou non liquide.

Par acide gras liquide, on entend un acide gras liquide à température ordinaire (25 °C) et à pression atmosphérique (760 mm de Hg, soit 1,013.10⁵ Pa).

Les acides gras de l'invention peuvent être saturés ou insaturés.

Les acides gras convenant à la mise en oeuvre de l'invention sont plus particulièrement choisis parmi les acides saturés ou insaturés, linéaires ou ramifiés, comportant de 8 à 30 atomes de carbone.

De préférence, l'acide gras est choisi parmi l'acide stéarique, l'acide palmitique, l'acide palmitoléique, l'acide linoléique, l'acide caprylique, l'acide myristique, l'acide laurique, l'acide béhénique et l'acide isostéarique.

### Sels métalliques

Les sels métalliques sont de préférence des hydroxydes, par exemple des hydroxydes d'aluminium ; des hydroxydes de métal alcalin comme le sodium ou le potassium ; ou bien des hydroxydes de métal alcalino-terreux comme le magnésium.

De préférence, le sel métallique est l'hydroxyde d'aluminium.

De préférence, l'agent de traitement de surface selon l'invention est un mélange d'acide gras et d'un sel métallique, de préférence un mélange d'acide stéarique et d'hydroxyde d'aluminium.

De préférence, le pigment minéral nanométrique traité en surface selon l'invention est le dioxyde de titane nanométrique traité par l'hydroxyde d'aluminium et l'acide stéarique, tel que celui commercialisé sous le nom MICRO TITANIUM DIOXIDE MT-100 T V par TAYCA.

### Milieu aqueux physiologiquement acceptable

La composition selon l'invention comprend un milieu aqueux physiologiquement acceptable. Ledit milieu comprend de l'eau.

L'eau utilisée peut être de l'eau déminéralisée stérile et/ou une eau florale telle que de l'eau de rose, de l'eau de bleuet, de l'eau de camomille ou de l'eau de tilleul, et/ou une eau thermale ou minérale naturelle.

La composition comprend de préférence au moins 5% en poids d'eau par rapport au poids total de la composition, de préférence au moins 10% en poids, de préférence au moins 20% en poids, de préférence au moins 30% en poids, de préférence au moins 40% en poids. La composition comprend de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50%.

La phase aqueuse peut également comprendre au moins un solvant organique miscible dans l'eau à 25°C.

De préférence, le solvant organique miscible dans l'eau est choisi parmi les alcools, les polyols et leurs mélanges.

Parmi les alcools, on peut citer les alcools en C₁-C₁₀, plus préférentiellement en C₁-C₅, tels que l'éthanol, l'isopropanol, le propanol et le butanol.

Le polyol est, de préférence, choisi parmi les polyols ayant de 2 à 20 atomes de carbone, plus préférentiellement de 2 à 6 atomes de carbone, comme le glycérol, le diglycérol, le propylèneglycol, l'isoprène glycol, le dipropylèneglycol, le butylène glycol, l'hexylène glycol, le 1,2-propanediol, le 1,3-propanediol, le pentylène glycol, les polyéthylèneglycols ayant de 2 à 200 motifs d'oxyde d'éthylène et leurs mélanges.

La composition peut comprendre de 0,1% à 25% en poids de solvant organique miscible dans l'eau, par rapport au poids total de la composition, plus préférentiellement de 0,5% à 20% en poids, encore plus préférentiellement de 0,9% à 15% en poids.

### Alpha hydroxy acide (AHA)

La composition selon l'invention peut comprendre au moins un AHA.

Par alpha hydroxy acide, on entend selon la présente invention un acide carboxylique ayant au moins une fonction hydroxy occupant une position alpha sur ledit acide (carbone adjacent à une fonction acide carboxylique). Cet acide peut se présenter dans la composition finale sous forme d'acide libre et/ou sous la forme de l'un de ses sels associés (sels avec une base organique ou un alcalin notamment), en particulier selon le pH final imposé à la composition.

Les α-hydroxyacides (alpha hydroxyacides ou AHA) sont par exemple choisis parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

Selon un mode préféré, l'alpha hydroxyacide est choisi parmi l'acide lactique, l'acide citrique, l'acide malique, l'acide tartrique et leurs sels. Plus particulièrement, l'alpha hydroxyacide est choisi parmi l'acide lactique, l'acide citrique, leurs sels et leurs mélanges.

Le ou les alpha hydroxyacides peuvent être présents en une quantité allant de 0,001 à 10% en poids, de 0,005 à 5% en poids, de préférence de 0,01 à 3% en poids par rapport au poids total de la composition.

### Tensioactifs

La composition peut comprendre au moins un tensioactif. Le tensioactif est de préférence choisi parmi les tensioactifs anioniques, cationiques, non-ioniques, zwitterioniques et amphotères. De préférence, il est choisi parmi :
a) les tensioactifs non ioniques, en particulier de HLB supérieure ou égale à 8 à 25°C, utilisés seuls ou en mélange. On peut citer notamment les esters d'acide gras et de polyglycérol, les esters d'acide gras et de polyéthylène glycol, les alkylC8-C30(poly)glycosides ou leurs mélanges. En particulier, le tensioactif non ionique peut être un ester d'acide gras comprenant de 10 à 18 atomes de carbone et de polyglycérol comprenant de 2 à 10 motifs de glycérol. Dans un mode de réalisation préféré de l'invention, l'ester d'acide gras comprend 12 atomes de carbone et 10 motifs de glycérol, et est le monolaurate de polyglycéryle comprenant 10 motifs de glycérol, i.e. le monolaurate de polyglycéryl-10 (disponible sous la dénomination commerciale DERMOFEEL G 10 L par la société Dr Straetmans). Il peut également être le monooléate de polyglycéryl-2 (disponible sous la dénomination commerciale SUNSOFT Q-17D(G)-C par la société TAIYO KAGAKU), ou bien le monocaprate de polyglycéryl-4 ou caprate de PG-4 (disponible sous la dénomination commerciale TEGOSOFT PC 41 par la société EVONIK GOLDSCHMIDT).
b) les tensioactifs anioniques tels que :
   les sels d'acides gras polyoxyéthylénés notamment ceux dérivant des sels alcalins, et leurs mélanges ;
   les esters phosphoriques et leurs sels tels que le "DEA oleth-10 phosphate" (Crodafos N 10N de la société CRODA) ou le phosphate de monocétyle monopotassique (Amphisol K de Givaudan) ;
   les sulfosuccinates tels que le "Disodium PEG-5 citrate lauryl sulfosuccinate" et le "Disodium ricinoleamido MEA sulfosuccinate" ;
   les alkyléthersulfates tels que le lauryl éther sulfate de sodium ;
   les iséthionates ;
   les acylglutamates tels que le "Disodium hydrogenated tallow glutamate" (AMISOFT HS-21 R^{®} commercialisé par la société AJINOMOTO) et le sodium stearoyl glutamate (AMISOFT HS-11 PF^{®} commercialisé par la société AJINOMOTO) et leurs mélanges ;
   les dérivés de soja comme le potassium soyate ;
   les citrates, comme le Glyceryl stéarate citrate (Axol C 62 Pellets de Degussa) ;
   les dérivés de proline, comme le Sodium palmitoyl proline (Sepicalm VG de Seppic), ou le Mélange de Sodium palmitoyl sarcosinate, Magnésium palmitoyl glutamate, Palmitic acid et Palmitoyl proline (Sepifeel One de Seppic) ;
   les lactylates, comme le Sodium stearoyl lactylate (Akoline SL de Karlshamns AB) ;
   les sarcosinates, comme le sodium palmitoyl sarcosinate (Nikkol sarcosinate PN) ou le mélange de Stéaroyl sarcosine et Myristoyl sarcosine 75/25 (Crodasin SM de Croda) ;
   les sulfonates, comme le Sodium C14-17 alkyl sec sulfonate (Hostapur SAS 60 de Clariant) ;
   les glycinates, comme le sodium cocoyl glycinate (Amilite GCS-12 d'Ajinomoto) ;
   les sels d'acides gras en C16-C30 notamment ceux dérivant des amines, comme le stéarate de triéthanolamine et/ou le stéarate d'amino-2-méthyl-2-propane di-ol-1,3 ;
c) les tensioactifs amphotériques comme les N-acyl-aminoacides tels que les N-alkyl-aminoacétates et le cocoamphodiacetate disodique et les oxydes d'amines tels que l'oxyde de stéaramine ou encore des tensioactifs siliconés comme les diméthicone copolyols phosphates tels que celui vendu sous la dénomination PECOSIL PS 100^{®} par la société PHOENIX CHEMICAL ;
et leurs mélanges.

### pH de la composition

La composition selon l'invention présente un pH inférieur ou égal à 7, de préférence inférieur ou égal à 6,5, de préférence inférieur ou égal à 6,3. Avantageusement, le pH de la composition est compris entre 3 et 6,3, de préférence compris entre 4 et 6,3.

De préférence, la composition cosmétique selon l'invention comprend au moins une base et/ou au moins un acide. La base et l'acide selon l'invention sont connus et classiquement utilisés dans le domaine cosmétique.

La base et/ou l'acide sont notamment utilisés pour ajuster le pH final de la composition entre 3 et 6,3.

L'acide peut être par exemple l'acide citrique.

La base peut être choisie parmi les bases minérales comme par exemple les hydroxydes de métaux alcalins, l'hydroxyde de sodium, l'hydroxyde de potassium.

De préférence, la base de la composition est un hydroxyde de métal alcalin, de préférence l'hydroxyde de sodium ou l'hydroxyde de potassium.

La composition selon l'invention peut comprendre au moins une base en une teneur en matière active allant de 0,5 % à 10 % en poids, par rapport au poids total de la composition, notamment de 1 % à 5 % en poids, de préférence allant de 1 % à 4 % en poids.

La composition conforme à l'invention peut être obtenue de manière classique par l'homme du métier.

Elle a également pour objet un procédé de maquillage et/ou de soin de la peau et/ou des phanères, dans lequel on applique la composition selon l'invention sur la peau et/ou les phanères.

Les exemples qui suivent permettront de mieux comprendre l'invention, sans toutefois présenter un caractère limitatif. Les matières premières sont nommées par leur nom chimique ou INCI. Les quantités indiquées sont en % en poids de matières premières par rapport au poids total de composition (% p/p), sauf mention contraire.

**Exemple 1 : préparation d'une composition selon l'invention et comparaison avec**

### des compositions comparatives

Les compositions A selon l'invention, et B comparative, ont été préparées par mélange des ingrédients du tableau 1.

La composition A selon l'invention contient du TiO2 traité en surface par de l'hydroxyde d'aluminium et de l'acide stéarique.

La composition comparative B ne contient pas de chitosan.

La composition A selon l'invention présente une bonne empreinte carbone notamment en favorisant l'emploi de matières premières renouvelables et/ou avec un bon index de naturalité et/ou d'origine naturelle et plus particulièrement d'origine végétale.

Les résultats sont dans le tableau 1.

**[Table 1]**

| **Ingrédients (% p/p)** | **A selon l'invention** | **B comparative** |
|---|---|---|
| Chitosan | 1 | - |
| Acide lactique | 0,5 | 0,5 |
| Glycérol | 1 | 1 |
| Polyglycéryl-10 laurate | 0,1 | 0,1 |
| Pigment de dioxyde de titane (TiO2) nanométrique traité par de l'hydoxyde d'aluminium et de l'acide stéarique (MICRO TITANIUM DIOXIDE MT-100 T V de TAYCA) | 15 | 15 |
| Eau | Qsp 100 | Qsp 100 |

Les formules ont été fabriquées selon le même procédé suivant :
L'acide lactique et le chitosan ont été mélangés sous rotor-stator de la marque VMI jusqu'à dissolution complète du chitosan. Le glycérol et le polyglyceryl-10 laurate ont été ajoutés sous faible agitation. Le TiO2 a ensuite été ajouté progressivement et maintenu sous agitation pendant 20 min.

### Evaluations :

### Macroscopiques :

Les échantillons ont été observés 24h après la fabrication pour déceler un éventuel déphasage des systèmes.

### De la tenue :

Afin d'évaluer la tenue à la friction à sec, à l'eau et à l'huile, 30 µl de formule ont été déposés sur 5×5 cm², sur des plaques noires. Chaque étalement a été fait au doigt en faisant 5 allers-retours, puis de légers cercles sur l'intégralité de la surface. Les dépôts sont séchés à l'air libre 3 heures. Ils ont ensuite été soumis à différents stress frictionnels :
- Friction à sec : chaque dépôt a été frictionné au doigt sur 15 allers-retours ;
- Friction à l'eau : chaque dépôt a été frictionné au doigt sur 15 allers-retours après dépôt d'eau ;
- Friction à l'eau + friction tissu sec : chaque dépôt a été frictionné au doigt sur 15 tours puis essuyé avec un tissu sec ;
- Friction à l'huile (OCTYLDODECANOL) + friction tissu sec : chaque dépôt a été frictionné au doigt sur 15 tours puis essuyé avec un tissu sec.

### Résultats :

### Macroscopiques :

Les échantillons ne présentent pas de déphasage. Le chitosan permet toutefois une meilleure stabilisation de la dispersion de TiO2.

### De la tenue :

L'ensemble des évaluations à la tenue des dépôts soumis à différentes sollicitations montrent une nette différence entre les échantillons avec et sans chitosan. Les dépôts avec chitosan restent intacts après sollicitation, tandis que les dépôts sans chitosan sont fortement dégradés.

L'ensemble des résultats est regroupé dans le tableau ci-dessous :

**[Table 2]**

| Formule | **A** | **B** |
|---|---|---|
| Présence/Absence Chitosan | Présent | Absent |

| **Note tenue (de 1 (-) à 5 (+))** | | |
|---|---|---|
| Friction sec | 4 | 2 |
| Friction Eau | 5 | 2 |
| Friction Eau + Tissu sec | 5 | 2 |
| Friction Huile + tissu sec | 4 | 1 |

| **Evaluation macroscopique** | | |
|---|---|---|
| Aspect du pot de stockage après 24h | Homogène | Déphasage |

La formule comparative sans chitosan B, en plus de présenter un déphasage rapide, montre en surface du TiO2 démouillé qui forme un nuage de poudre potentiellement inhalable.

### Exemple 2 : évaluation du FPS d'une composition selon l'invention et comparaison avec une composition comparative

L'idée est de comparer le Facteur de Protection Solaire (FPS ou SPF en anglais, Sun Protection Factor) *in vitro* d'une formule aqueuse selon l'invention versus une formule huileuse comparative, avec le même TiO2 en même quantité.

Les deux formules testées ont été préparées comme décrit dans l'exemple 1.

**[Table 3]**

| **Ingrédient** | **Formule comparative** | **Formule selon l'invention** |
|---|---|---|
| Eau | | Qsp 100 |
| Glycérol | | 1 |
| Pigment de dioxyde de titane (TiO2) nanométrique traité par de l'hydoxyde d'aluminium et de l'acide stéarique (MICRO TITANIUM DIOXIDE MT-100 T V de TAYCA) | 15 | 15 |
| ISONONYL ISONONANOATE | 83,5 | |
| Acide polyhydroxystéarique | 0,75 | |
| POLYGLYCERYL-10 LAURATE | | 0,1 |
| POLYGLYCERYL-6 POLYRICINOLEATE | 0,75 | |
| Acide lactique | | 0,5 |
| CHITOSAN | | 1 |

### Evaluation in vitro du SPF

### Protocole d'évaluation l'efficacité filtrante

In-vitro : Le facteur de protection solaire (SPF) est déterminé selon la méthode « in vitro » décrite par M. Pissavini et al dans International Journal of Cosmetic Science, 40, 263-268 (2018), sur la base de l'absorbance initiale.

Le facteur de protection UVA (UVAPF) in vitro d'un produit de protection solaire contre le rayonnement UVA est calculé mathématiquement par modélisation spectrale in vitro selon le protocole ISO 24443 :2012 (Fr).

Chaque composition est appliquée sur six plaques rugueuses de PMMA, sous la forme d'un dépôt homogène et régulier à raison de 1 mg/cm2. L'étalement de chaque composition se fait à l'aide d'un robot automatisé qui fait des mouvements réguliers et uniformes sur trois plaques dit HD6 (plaques granuleuse moulé) et trois plaques dit SB6 (plaques granuleuse sablé). La plaque est pesée avant et après étalement. Une fois que les six plaques sont étalées, elles sont mises au repos dans des Thermo-Master dans le noir à 25 °C pendant 30 minutes. Les mesures sont réalisées grâce au spectrophotomètre UV-1000S de la société Labsphère. Neuf mesures par plaque sont effectuées, puis sont analysées à l'aide un tableur Excel fournissant les valeurs du SPF et UVAPF de la composition mesurée.

**[Table 4]**

| | **SPF in vitro** | Ecart-type (SPF DA) | **UVAPF** | Ecart-type (UVA DA) |
|---|---|---|---|---|
| **Formule comparative** | **46,3** | 3,9 | **8,3** | 0,5 |
| **Formule selon l'invention** | **67,8** | 7 | **8,35** | 0,65 |

Les résultats montrent un gain de 46,4 % de SPF *in vitro* avec la formule selon l'invention.

**Conclusion :** une composition selon l'invention permet de booster le SPF par rapport à une composition huileuse comparative contenant le même TiO2 en même quantité.

## Revendications

1. Composition cosmétique, notamment de maquillage et/ou de soin de la peau et/ou des lèvres, comprenant, dans un milieu aqueux physiologiquement acceptable :
a) au moins 0,01% en poids par rapport au poids total de composition de chitosan natif ayant un poids moléculaire strictement supérieur à 3000 Daltons, cette quantité étant strictement inférieure à 15% en poids, et
b) au moins un pigment minéral traité en surface, ledit pigment présentant une taille inférieure ou égale à 100 nm.

2. Composition selon la revendication 1, dans laquelle le chitosan natif a un poids moléculaire supérieur ou égal à 10 kDa, de préférence supérieur ou égal à 15 kDa, de préférence supérieur ou égal à 20 kDa, de préférence le chitosan natif a un poids moléculaire compris entre 10 kDa et 2 MDa, de préférence compris entre 15 kDa et 1,5 MDa, de préférence compris entre 20 kDa et 300 kDa, de préférence compris entre 20 kDa et 200 kDa.

3. Composition selon la revendication 1 ou 2, dans laquelle le chitosan natif a un degré d'acétylation du chitosan inférieur ou égal à 80%, de préférence inférieur ou égal à 70%, de préférence inférieur ou égal à 60%, de préférence inférieur ou égal à 50%, de préférence inférieur ou égal à 35%, de préférence inférieur ou égal à 25%, de préférence inférieur ou égal à 15%.

4. Composition selon l'une des revendications précédentes, dans laquelle le chitosan natif est un polysaccharide préparé à partir d'une origine fongique, de préférence issu du mycélium d'un champignon du type Ascomycète, et en particulier *d'Aspergillus niger* et/ou d'un champignon Basidiomycète, et en particulier *Lentinula edodes* et/ou *Agaricus bisporus,* de préférence le champignon est *Aspergillus niger.*

5. Composition selon l'une des revendications précédentes, dans laquelle le chitosan natif est présent en une quantité allant de 0,01% à 14% en poids, de préférence de 0,1% à 14% en poids, de préférence de 0,1% à 12% en poids, de préférence de 0,2% à 7% en poids, de préférence de 0,25% à 5% en poids par rapport au poids total de la composition.

6. Composition selon l'une des revendications précédentes, qui comprend de 1% à 70% en poids, de préférence de 1 à 60% en poids, de préférence de 1% à 50% en poids, de préférence de 3% à 45% en poids, par rapport au poids de la composition, de préférence de 4% à 30% en poids, de préférence de 6% à 20% en poids, de préférence de 6% à 15% en poids, de pigment(s) minéral traité(s).

7. Composition selon l'une des revendications précédentes, dans laquelle le pigment minéral est le dioxyde de titane.

8. Composition selon l'une des revendications précédentes, dans laquelle le pigment minéral présente une taille **caractérisée par** un D[50] inférieur à 100 nm, de préférence inférieur à 50 nm, de préférence compris entre 5 et 40 nm, de préférence compris entre 10 et 30 nm, où D[50] représente la taille maximale que présentent 50 % en volume des particules.

9. Composition selon l'une des revendications précédentes, dans laquelle le pigment est traité par un agent de traitement de surface hydrophobe, de préférence choisi parmi les acides gras, les sels métalliques et leurs mélanges ; de préférence l'agent de traitement de surface est un mélange d'acide gras et d'un sel métallique, de préférence un mélange d'acide stéarique et d'hydroxyde d'aluminium.

10. Composition selon l'une des revendications précédentes, dans laquelle le pigment traité en surface est le dioxyde de titane traité par l'hydroxyde d'aluminium et l'acide stéarique.

11. Composition selon l'une des revendications précédentes, dans laquelle le milieu aqueux physiologiquement acceptable comprend au moins 5% en poids d'eau par rapport au poids total de la composition, de préférence au moins 10% en poids, de préférence au moins 20% en poids, de préférence au moins 30% en poids, de préférence au moins 40% en poids ; de préférence de 5% à 95% en poids d'eau par rapport au poids total de la composition, plus préférentiellement de 10% à 85%, encore plus préférentiellement de 20% à 80%, encore plus préférentiellement de 25% à 70%, encore plus préférentiellement de 28% à 60%, encore plus préférentiellement de 30% à 50% ; et éventuellement au moins un solvant organique miscible dans l'eau à 25°C choisi parmi les alcools, les polyols et leurs mélanges.

12. Composition selon l'une des revendications précédentes, qui comprend au moins un alpha hydroxy acide, de préférence choisi parmi l'acide lactique, l'acide citrique, l'acide méthyllactique, l'acide glucuronique, l'acide glycolique, l'acide pyruvique, l'acide 2-hydroxy-butanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxy-nonanoïque, l'acide 2-hydroxydécanoïque, l'acide 2-hydroxyundécanoïque, l'acide 2-hydroxydodécanoïque, l'acide 2-hydroxytétradécanoïque, l'acide 2-hydroxy-hexadécanoïque, l'acide 2-hydroxyoctadécanoïque, l'acide 2-hydroxytétra-cosanoïque, l'acide 2-hydroxyeïcosanoïque ; l'acide mandélique ; l'acide phényllactique ; l'acide gluconique ; l'acide galacturonique ; l'acide aleuritique ; l'acide ribonique ; l'acide tartronique ; l'acide tartrique ; l'acide malique ; l'acide fumarique ; leurs sels et leurs mélanges.

13. Composition selon l'une des revendications précédentes, qui présente un pH inférieur ou égal à 7, de préférence inférieur ou égal à 6,5, de préférence inférieur ou égal à 6,3, de préférence compris entre 3 et 6,3, de préférence compris entre 4 et 6,3.

14. Composition selon l'une des revendications précédentes qui est substantiellement exempte de polymère (méth)acrylique et/ou de silicone ; de préférence la composition comprend moins de 1% en poids par rapport au poids total de la composition, de préférence moins de 0,5% en poids, de préférence moins de 0,3% en poids, préférentiellement 0,1% en poids de polymère (méth)acrylique, de préférence la composition est totalement exempte de polymère (méth)acrylique ; de préférence la composition comprend moins de 1% en poids par rapport au poids total de la composition, de préférence moins de 0,5% en poids, de préférence moins de 0,3% en poids, préférentiellement 0,1% en poids de silicone, de préférence la composition est totalement exempte de silicone.

15. Composition selon l'une des revendications précédentes, **caractérisée en ce que** le ratio pondéral entre le pigment minéral nanométrique traité en surface et le chitosan (i.e. ratio pondéral pigment : chitosan) est supérieur à 1, de préférence compris entre 1 et 30, de préférence compris entre 1 et 25, préférentiellement entre 5 et 20.

16. Procédé de maquillage et/ou de soin de la peau et/ou des phanères, dans lequel on applique la composition selon l'une des revendications précédentes sur la peau et/ou les phanères.
